# EUROPÄISCHE PATENTSCHRIFT

(11) **EP 2 815 728 B1**
(45) Veröffentlichungstag und Bekanntmachung des Hinweises auf die Patenterteilung: **06.01.2016**
(21) Anmeldenummer: 14166213.0
(22) Anmeldetag: 28.04.2014
(51) Int. Cl.: A61F 2/78

(54) **Anordnung zum Überziehen über einen Gliedmaßenstumpf zur Fixierung eines Prothesenschafts**
Assembly for drawing over a limb stump for fixing a prosthetic shaft
Système de recouvrement d'un manchon de réception de moignon destiné à fixer une tige de prothèse

(30) Priorität: 21.06.2013 DE 102013010371
(43) Veröffentlichungstag der Anmeldung: 24.12.2014
(73) Patentinhaber: medi GmbH & Co. KG, 95448 Bayreuth (DE)
(72) Erfinder: Kurth, Christof, 95500 Heinersreuth (DE)
(74) Vertreter: Lindner Blaumeier Patent- und Rechtsanwälte

(56) Entgegenhaltungen:
- EP-A2- 2 068 775
- DE-A1-102007 035 409
- US-A- 6 059 834
- US-A1- 2005 267 599
- US-A1- 2010 274 364
- US-A1- 2012 095 571

## Beschreibung

Die Erfindung betrifft eine Anordnung zum Überziehen über einen Gliedmaßenstumpf zur Fixierung eines Prothesenschafts, umfassend einen über den Stumpf zu ziehenden Liner und einen über diesen außenseitig zu ziehenden Gestricküberzug zur Kompensation von Stumpfvolumenschwankungen, wobei der Liner wenigstens eine im Bereich des distalen Endes ringförmig umlaufende Dichtlippe zur dichten Anlage an der Innenseite des Prothesenschaftes aufweist.

Zur Fixierung eines Prothesenschaftes beispielsweise einer Unterschenkel- oder Oberschenkelprothese kommen zunehmend sogenannte Silikonliner zum Einsatz, zumindest außenseitig aus Silikon, gegebenenfalls mit Gleitbeschichtung, bestehend und an ihrem distalen Ende üblicherweise mehrere umlaufende Dichtlippen aufweisen, die sich beim Einsteigen in den Prothesenschaft an die Schaftinnenseite anlegen und so den distalen Schaftbereich abdichten. Über ein schaftseitig vorgesehenes Ventil wird Luft aus diesem Bereich nach außen gedrückt oder es wird über eine mechanische oder elektrische Pumpe Luft aus diesem Bereich abgesaugt, so dass die Fixierung des Schaftes über den Liner durch Unterdruck sichergestellt ist. Der Prothesenschaft ist in seiner Geometrie respektive dem umschlossenen Volumen im Wesentlichen dem Volumen des Stumpfes mit angelegtem Liner angepasst, so dass der Stumpf hinreichend fest und spielfrei im Schaft aufgenommen ist. Mitunter kommt es jedoch zu Schwankungen des Stumpfvolumens, das heißt, dass der Stumpf umfangsmäßig etwas abnimmt, so dass ein satter Sitz im Schaft nicht mehr unbedingt gegeben ist. Um solche Stumpfvolumenschwankungen zu kompensieren ist es bekannt, über den Liner einen Gestricküberzug zur Kompensation zu ziehen, der den Gesamtumfang im Stumpfbereich wieder etwas vergrößert, so dass die Volumenabnahme hierüber ausgeglichen und der Stumpf wieder hinreichend stabil im Schaft aufgenommen ist.

Um zu verhindern, dass der Gestricküberzug beim Einsteigen in den Prothesenschaft nach oben rutscht oder sich aufrollt, wird im Bereich des distalen Endes des Gestricküberzugs ein elastisches Bündchen vorgesehen, das beim Überziehen des Gestricküberzugs über den angelegten Silikonliner distal gesehen über die oberste Dichtlippe gezogen wird, mithin also, gesehen in Einstiegrichtung, unterhalb der obersten Dichtlippe angeordnet ist und sich im Bereich unter der Dichtlippe zusammenzieht. Hierüber wird der Gestricküberzug unterhalb dieser Dichtlippe, die als "Arretierung" dient, quasi festgezogen. Auch kann der Überzug unter die Dichtlippe geschoben werden.

Daneben kommen Liner mit einer oder mehreren Dichtlippen zum Einsatz, die außenseitig aus Silikon bestehen und einen äußeren Textilüberzug aufweisen. Auch hier ist die Handhabung die gleiche, ein Gestricküberzug wird, um sein Hochrutschen zu verhindern, über die Dichtlippe gezogen oder unter sie geschoben.

Ferner sind Liner aus Polyurethan bzw. mit einer Polyurethanaußenfläche, gegebenenfalls mit einer auch nur lokal aufgetragenen Gleitbeschichtung oder einem Textilüberzug bekannt, die auch eine oder mehrere Dichtlippen aufweisen. Die Handhabung ist auch hier wie oben beschrieben.

Nachteilig hierbei ist, dass der über diese oberste Dichtlippe gezogene oder unter sie geschobene Gestricküberzug den Durchmesser respektive Umfang im Bereich dieser Dichtlippe erhöht. Hieraus erhöht sich letztlich der auf den Stumpf wirkende Druck im Bereich dieser Dichtlippe zusätzlich. Die Dichtlippe selbst ist materialbedingt in ihrer radialen Dehnbarkeit eingeschränkt, der notwendige Anpressdruck der Dichtlippe gegen den Schaft wirkt auch als zusätzliche Druckkomponente unterhalb der Dichtlippe auf den Stumpf. Wird nun über den Gestricküberzug der Durchmesser respektive Umfang im Bereich dieser Dichtlippe noch weiter erhöht, nimmt der Druck auf den Stumpf lokal zu. Der vom Liner auf den Stumpf ausgeübte Druck sollte jedoch von distal nach proximal bevorzugt abnehmen, da ansonsten keine zuverlässige Entstauung des Stumpfes gewährleistet werden kann. Eine den Stumpf umlaufende, ringförmige Zone erhöhten Drucks kann somit im Extremfall zu einer Ödemisierung der Bereiche des Stumpfes distal dieser Zone führen, was beim Anwender zu gesundheitlichen Beeinträchtigungen führen kann, insbesondere wenn zusätzliche Erkrankungen wie arterielle Verschlusskrankheit oder venöse Degeneration oder Kreislaufinsuffizienz, wie sie beispielsweise bei Diabetes häufig vorliegen, gegeben sind.

Aus US 2012/095571 A1 ist ein Liner bekannt, der eine integrierte, der Volumenanpassung dienende Einlage aufweist, über die eine Anpassung des Gliedmaßenstumpfes an den Prothesenschaft möglich ist. Diese Einlage findet sich in dem Bereich, in dem mehrere radial nach außen abstehende Dichtlippen an der Lineraußenseite vorgesehen sind.

Aus EP 2 068 775 A2 ist ein Liner bekannt, an dessen Außenseite ebenfalls mehrere radial abstehende Dichtlippen unterschiedlicher Geometrie vorgesehen sind. Diese Dichtlippen können auch mittels separater, an der Lineraußenseite anbringbarer Elemente realisiert werden.

US 2005/0267599 A1 beschreibt einen Liner, an dessen Außenseite eine Vielzahl ringförmig umlaufender, nach außen gewölbter Dichtelemente vorgesehen sind, wobei zusätzlich auch axial verlaufende, ringförmige Vorsprünge an der Außenseite realisiert sein können.

DE 10 2007 035 409 A1 offenbart ein orthopädisches Interface in Form eines Liners, mit einem flächigen 3D-Textil mit einer Oberseite und einer Unterseite, die über Stützfäden voneinander beabstandet gehalten und miteinander verbunden sind. Die Unterseite dieses 3D-Abstandsgewirkes ist mit einer auf der Haut des Trägers haftenden Beschichtung versehen.

Aus US 6,059,834 A ist ein schlauchförmiger Überzug zum Überziehen über den Prothesenstrumpf bekannt, der aus einem dehnbaren Material besteht. Die Innenseite dieses Überzugs ist mit einem Anti-Rutsch-Material belegt, vorzugsweise bestehend aus einem synthetischen Harzmaterial.

Schließlich ist aus US 2010/0274364 A1 ein Prothesensystem bekannt, mit einem Prothesenschaft zur Aufnahme des Gliedmaßenstumpfes, wobei die Form oder Geometrie des Prothesenschaftes zur Verbesserung der Stumpfhalterung veränderbar ist.

Der Erfindung liegt damit das Problem zugrunde, eine Anordnung anzugeben, die einerseits eine Stumpfvolumenkompensation unter Verwendung eines Gestricküberzugs ermöglicht, gleichzeitig aber keine Nachteile für den Anwender, insbesondere im Hinblick auf Druckerhöhungen auf den Stumpf, mit sich bringt.

Zur Lösung dieses Problems ist bei einer Anordnung der eingangs genannten Art erfindungsgemäß vorgesehen, dass der Gestricküberzug lediglich im Bereich seines distalen Endes eine Haftschicht aufweist, die adhäsiv mit der Lineroberfläche zusammenwirkt.

Erfindungsgemäß wird der Gestricküberzug über eine an ihm vorgesehene Haftschicht unmittelbar auf der Oberfläche des Liners adhäsiv fixiert. Das heißt, dass der gesamte Gestricküberzug ausschließlich flächig auf der Lineroberfläche von distal nach proximal verläuft. Die Lineroberfläche kann dabei eine Silikon- oder Polyurethanoberfläche sein, sie kann von einer, gegebenenfalls auf eine solche Silikon- oder Polyurethanoberfläche aufgetragenen, sehr dünnen Gleitbeschichtung gebildet sein, oder von einem Textilüberzug, der die Außenhaut des Liners bildet. Zu all diesen unterschiedlichen Oberflächen geht die nur lokal im Bereich eines Endes vorgesehene Haftschicht eine adhäsive Verbindung ein und fixiert den Gestricküberzug. Der Gestricküberzug verläuft ausschließlich proximal der obersten Dichtlippe, er steht mit dieser nicht in Kontakt, so dass es im Dichtlippenbereich zu keiner Umfangs- oder Durchmesservergrößerung kommt. Vielmehr kann die Dichtlippe ihre Dichtfunktion, die sie bei bisher bekannten Systemen infolge des Übergriffs des Gestricküberzugs nicht mehr ausführen konnte, bei der erfindungsgemäßen Anordnung leisten, das heißt, dass wiederum sämtliche Dichtlippen an der Abdichtung zum Prothesenschaft hin teilnehmen, so dass für den Anwender der Schaft in gleicher Weise wie ohne Gestricküberzug fixiert ist.

Die Überzugfixierung erfolgt adhäsiv, indem die Haftschicht entsprechend mit der Lineroberfläche zusammenwirkt, sei sie aus Silikon oder Polyurethan, aus einer Gleitbschichtung oder aus einem Textil, so dass sich quasi eine pseudo-klebrige Haftverbindung ergibt, die hinreichend stabil ist, dass sich der Gestricküberzug beim Einsteigen, wenn quasi eine Kraft mit einer in Längsrichtung des Überzugs gerichteten Komponente wirkt, nicht löst und der Überzug nicht auf der Silikon/Polyurethan-, Gleitbeschichtungs- oder Textilfläche des Liner abrutscht.

Die Haftschicht selbst ist bevorzugt lediglich auf der Innenseite des Gestricküberzugs aufgebracht, das heißt, dass sie nur direkt auf der Gestrickoberfläche vorgesehen ist, sie kann aber auch teilweise in das Gestrick eingedrungen sein, je nachdem, wie die Haftschicht ausgelegt respektive aufgebracht wird.

Nach einer ersten Erfindungsalternative kann die Haftschicht in Form eines Haftbandes aufgenäht oder aufgeklebt sein. In diesem Fall ist die Haftschicht lediglich oberflächlich am Gestricküberzug vorgesehen. Alternativ kann die Haftschicht auch auf das Gestrick aufgegossen, aufgestrichen oder auf das Gestrick aufgeschmolzen und ausgehärtet respektive vernetzt sein. In diesem Fall ist die Haftschicht mittels eines zunächst fluiden Materials aufgebracht, was durch Aufgießen oder Aufstreichen erfolgen kann, wonach durch Erwärmen mittels UV- oder IR-Bestrahlung oder Ähnlichem die Haftschicht aushärtet respektive vernetzt. Sie kann auch durch Aufschmelzen und anschließendes Aushärten respektive Vernetzen aufgebracht werden. Hierzu wird zunächst ein Haftband aufgelegt, das sodann durch Erwärmung, beispielsweise Hochfrequenz- oder Ultraschallbeheizung, direkte Beheizung unter Verwendung erwärmter Platten oder Ähnlichem aufgeschmolzen wird, so dass es das Gestrick benetzt (wie bei der Aufstreich- oder Aufgießlösung) und sich eine entsprechende Verbindung respektive ein entsprechendes Eindringen in das Gestrick ergibt. Anschließend erfolgt auch hier das Aushärten respektive Vernetzen, so dass die Haftschicht sicher am Gestrick fixiert ist.

Bevorzugt wird als Haftschichtmaterial ein dehnbares Elastomer oder ein Elastomergel, das aus einem solchen Elastomer und einem Weichmacheröl besteht und hinreichend vernetzt ist, verwendet, wobei sich insbesondere die Verwendung eines Styrolblockcopolymers, sei es als reines Polymer oder als Gelkomponente, als besonders zweckmäßig im Hinblick auf eine gute adhäsive Verbindung insbesondere zur Silikon/Polyurethan- oder Gleitschichtoberfläche des Liners ergibt. Als besonders bevorzugt wird als Elastomer ein Styrol-Ethylen-Propylen-Styrol (SEPS) verwendet. SEPS weist eine hohe Adhäsivität bezüglich der Lineroberfläche auf, verbunden mit einer guten Beständigkeit gegenüber wässrigen Lösungen, anorganischen Säuren und Laugen, was es ermöglicht, den Gestricküberzug ohne weiteres auch waschen zu können. Auch ist eine hinreichende Temperaturbeständigkeit gegeben, so dass auch ein Waschen bei erhöhter Temperatur bis wenigstens 40° ohne weiteres möglich ist. Auch weist SEPS ein sehr gutes Alterungsverhalten auf, so dass eine lange Verwendung des Gestricküberzugs gewährleistet ist. Ferner ist es physiologisch unbedenklich und ohne weiteres hinsichtlich des Härtebereichs entsprechend einstellbar, was gleichermaßen für die mechanischen Werte hinsichtlich einer hohen Rissfestigkeit gilt. Insbesondere ein solches Styrolblockcopolymer, vorzugsweise in Form des SEPS, bietet gerade auf einer Silikonoberfläche oder einer Gleitbeschichtungsfläche, die besonders glatt sind und auf denen ein textiler Gestricküberzug extrem rutscht, eine sehr gute adhäsive Haftung.

Um zu vermeiden, dass durch Aufbringen der Haftschicht, insbesondere im Falle des Aufnähens oder Aufklebens, der Gestricküberzug in diesem Bereich dicker wird, sieht eine zweckmäßige Weiterbildung der Erfindung vor, dass der Gestricküberzug zumindest in dem Bereich, in dem die Haftschicht aufgebracht ist, dünner gestrickt ist als im übrigen Gestrickbereich. Dies ist ohne weiteres beim Stricken, z. B. Flachstricken des Gestricküberzugs möglich. Hierüber kann also die vom Haftschicht herrührende Aufdickung des Gestricks kompensiert werden, so dass das Gestrick über seine gesamte Länge nahezu gleiche Dicke aufweist.

Der Gestricküberzug weist zweckmäßigerweise eine zylindrische oder konische Grundform auf, das heißt, dass das z. B. als Flachgestrick hergestellte Gestrick dementsprechend gestrickt wird, dass es die entsprechende Form aufweist. Grundsätzlich denkbar wäre es aber auch, das Gestrick als Rundgestrick auszuführen.

Neben der Anordnung umfassend den Silikonliner und den Gestricküberzug betrifft die Erfindung ferner einen Gestricküberzug für eine solche Anordnung. Dieser Gestricküberzug zeichnet sich dadurch aus, dass lediglich im Bereich seines distalen Endes eine Haftschicht vorgesehen ist, die derart ausgelegt ist, dass sie adhäsiv mit einer Lineroberfläche bestehend aus Silikon oder Polyurethtan, einer, insbesondere auf der Silikon- oder Polyurethtanoberfläche aufgebrachten, Gleitbeschichtung oder einem textilen Überzug eines Liners zusammenwirkt. Das heißt, dass in der Tragstellung eine adhäsive Haftverbindung zwischen der Haftschicht und der Silikon/Polyurethtan-, Gleitbeschichtungs- oder Textiloberfläche gegeben ist, die es verhindert, dass der Gestricküberzug längs des Liners verrutschen kann, wenn der Träger in den Prothesenschaft steigt.

Die Haftschicht kann lediglich auf der Innenseite des Überzugs aufgebracht sein, mithin also oberflächlich beispielsweise in Form eines Haftbands aufgenäht oder aufgeklebt sein. Denkbar ist es aber auch, dass die Haftschicht teilweise in das Gestrick eingedrungen ist, beispielsweise indem die Haftschicht durch Aufgießen oder Aufstreichen eines fluiden Haftmaterials und anschließendes Aushärtens aufgebracht ist, oder indem ein Haftband aufgeschmolzen wird, so dass das fluide Haftmaterial in das Gestrick eindringen kann, wonach es aushärtet.

Zweckmäßigerweise besteht die Haftschicht aus einem dehnbaren Elastomer oder einem Elastomergel bestehend aus einem Elastomer und einem Weichmacheröl, wobei als Elastomer vorzugsweise ein Styrolblockcopolymer verwendet wird. Besonders bevorzugt ist als Elastomer Styrol-Ethylen-Propylen-Styrol-Copolymer (SEPS) verwendet.

Der Gestricküberzug sollte vorzugsweise zumindest in dem Bereich, in dem die Haftschicht aufgebracht ist, dünner gestrickt sein als im übrigen Gestrickbereich, um einen Dickenzuwachs durch Aufbringen der Haftschicht zu vermeiden.

Bevorzugt weist der Gestricküberzug eine konische Grundform auf, er kann aber auch zylindrisch sein. Er kann als Rundgestrick hergestellt werden, oder vorzugsweise als Flachgestrick.

Ferner betrifft die Erfindung ein Verfahren zur Herstellung eines Gestricküberzugs der vorbeschriebenen Art. Dieses Verfahren zeichnet sich dadurch aus, dass nach dem Stricken eines Gestricks, vorzugsweise eines Flachgestricks lediglich an einem Ende des Gestricks auf die Gestrickinnenseite eine adhäsive Haftschicht aus einem dehnbaren Elastomer oder einem Elastomergel bestehend aus einem Elastomer und einem Weichmacheröl in Form eines Haftbands aufgenäht oder aufgeklebt wird, oder durch Aufgießen, Aufstreichen oder Aufschmelzen und Aushärten mit dem Gestrick verbunden wird, wobei das Elastomer ein Styrolblockcopolymer ist. Wird ein Haftband verwendet, so kann dieses z.B. aus dem reinen Elastomer bestehen, das in Bandform vorliegt, es kann aber auch aus einem Trägerband, auf das eine Elastomer- oder Elastomergelbeschichtung aufgebracht ist, bestehen.

Als Elastomer wird verfahrensgemäß bevorzugt SEPS verwendet.

Das Styrolblockcopolymer, insbesondere das SEPS, wird vorzugsweise in Form eines Bandes auf das Flachgestrick aufgebracht und mittels Hochfrequenz- oder Ultraschallbeheizung oder mittels geheizter Platten erwärmt und aufgeschmolzen, so dass es das Flachgestrick benetzt, wonach es abkühlt und aushärtet.

Das Gestrick wird zumindest in dem Bereich, in dem die Haftschicht aufgebracht ist, dünner gestrickt als im übrigen Gestrickbereich, um eine Aufdickung durch die Haftschicht zu vermeiden. Das Gestrick, sei es ein Flachgestrick oder ein Rundgestrick, wird derart gestrickt, dass der Gestricküberzug entweder zylindrisch oder konisch ist.

Weitere Vorteile, Merkmale und Einzelheiten der Erfindung ergeben sich aus dem im Folgenden beschriebenen Ausführungsbeispiel sowie anhand der Zeichnungen. Dabei zeigen:
- Fig. 1: eine Prinzipdarstellung einer erfindungsgemäßen Anordnung umfassend einen erfindungsgemäßen Gestricküberzug, wobei Liner und Gestricküberzug separat voneinander gezeigt sind,
- Fig. 2: die Anordnung aus Fig. 1 mit über den Liner gezogenem Gestricküberzug,
- Fig. 3: eine Schnittansicht durch einen Abschnitt der Anordnung aus Fig. 2 im Bereich der adhäsiven Haftverbindung zwischen Gestricküberzug und Liner, und
- Fig. 4: eine vergrößerte Teilansicht des Gestricküberzugs im distalen Endbereich mit der aufgebrachten Haftschicht.

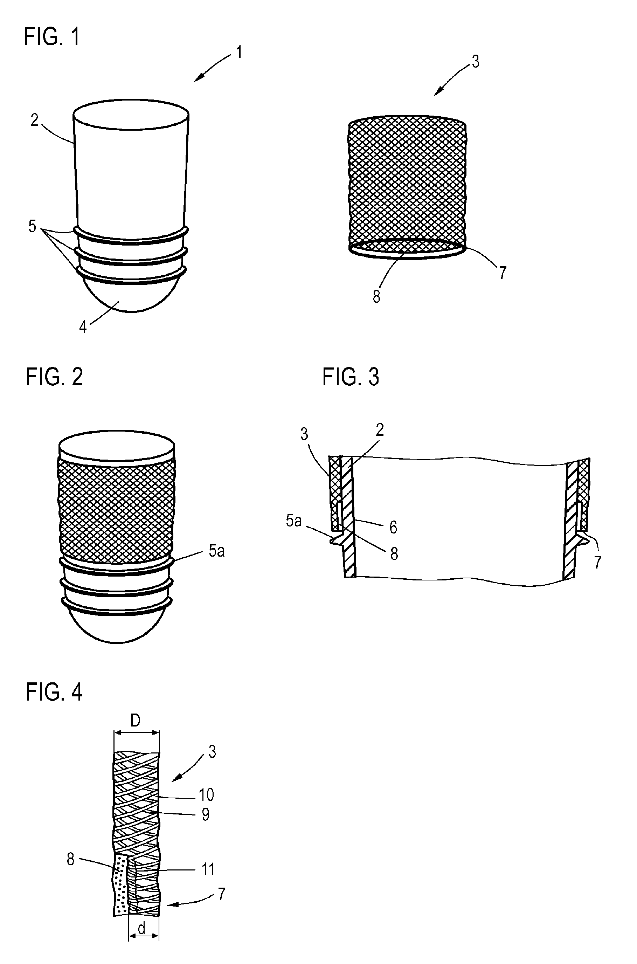

Fig. 1 zeigt eine erfindungsgemäße Anordnung 1 umfassend exemplarisch einen Silikonliner 2 und einen erfindungsgemäßen Gestricküberzug 3. Der Silikonliner 2 weist eine zylindrische oder konische Form auf, am distalen Ende 4 ist er kalottenförmig geschlossen. In diesem Bereich sind im gezeigten Ausführungsbeispiel insgesamt drei ringförmig umlaufende, radial nach außen vorspringende Dichtlippen 5, ebenfalls aus Silikon und damit flexibel, vorgesehen. Der Silikonliner 2, der auch mehrlagig aufgebaut sein kann, weist im gezeigten Ausführungsbeispiel eine Silikonoberfläche 6 auf, die gegebenenfalls mit einer Gleitbeschichtung versehen sein kann.

Der erfindungsgemäße Gestricküberzug 3, der vorzugsweise aus einem Flachgestrick besteht, weist im gezeigten Ausführungsbeispiel eine im Wesentlichen zylindrische Grundform auf, kann aber auch leicht konisch sein. Im Bereich seines distalen Endes 7 ist an der Gestrickinnenseite eine Haftschicht 8 vorgesehen, die adhäsiv mit der Lineroberfläche 6, sei es der blanken Silikonfläche, sei es der Gleitbeschichtungsfläche, zusammenwirkt, so dass sich eine adhäsive Haftverbindung zwischen der Haftschicht 8 und der Oberfläche 6 ergibt, wenn, wie in Fig. 2 gezeigt, der Gestricküberzug 3 über den (dann natürlich bereits über den Stumpf des Trägers gezogenen) Silikonliner gezogen ist. Der Gestricküberzug 3 dient dazu, etwaige Volumenschwankungen des Stumpfes zu kompensieren, so dass der Stumpf trotz abnehmendem Volumen noch sicher und fest im Prothesenschaft aufgenommen ist.

Die Haftschicht 8 bewirkt, dass beim Einsetzen des Stumpfes in den Prothesenschaft das untere Ende des Gestricküberzugs 3 nicht nach proximal verrutschen kann. Selbst wenn der Gestricküberzug 3 beim Einschieben an der Prothesenschaftinnenwand entlanggleitet und es folglich zur Reibung kommt, sind diese Reibungskräfte nicht ausreichend, den adhäsiven Haftverbund zwischen Haftschicht 8 und Lineroberfläche 6 zu lösen, so dass der Gestricküberzug 3 nicht längs des Liners 2 nach oben rutschen kann.

Als Haftschicht 8 wird vorzugsweise ein Elastomer verwendet, das hinreichend dehnbar ist, wenn es auf die Gestrickinnenseite aufgebracht und vernetzt ist. Bevorzugt kommt ein Styrolblockcopolymer, besonders bevorzugt SEPS, zum Einsatz. Dieses wird in Form eines Haftbandes auf die Gestrickinnenseite (vorzugsweise wenn das Flachgestrick noch nicht zur Schlauchform verbunden ist) gelegt, wonach es erwärmt wird, also aufgeschmolzen wird. Das dann fluide Elastomer respektive SEPS benetzt das Gestrick respektive die einzelnen Gestrickfäden, und dringt vorzugsweise etwas in das Gestrick ein. Anschließend wird das Elastomer abgekühlt, so dass es vernetzt und einen festen Verbund zum Textil eingeht.

Die Erwärmung kann mittels eines beliebigen Heizelements erfolgen, solange ein hinreichender Energieeintrag möglich ist. Zu nennen ist exemplarisch eine Hochfrequenz- oder Ultraschallbeheizung oder geheizte Platten, zwischen die das Gestrick nebst aufgelegtem Haftband gelegt wird und Ähnliches.

Alternativ kann auch ein Elastomergel bestehend aus einer Elastomerkomponente und einer Weichmacherkomponente, insbesondere einem Weichmacheröl, verwendet werden. Ein solches Elastomergel besitzt eine weiche Konsistenz, verbunden mit einer hohen Adhäsivität bzw. "Klebrigkeit", die für einen sehr guten adhäsiven Haftverbund zur Lineroberfläche sorgt. Auch hier wird als Elastomer vorzugsweise ein Styrolblockcopolymer, insbesondere SEPS, verwendet. Das Elastomergel wird vorzugsweise in flüssigem oder zähflüssigen Zustand aufgetragen, wonach es definiert vernetzt bzw. aushärtet und einen über eine Zusammensetzung definierten weichen Endzustand einnimmt. Natürlich kann ein solches Elastomergel auch als Haftband vorgefertigt aufgebracht werden. In diesem Fall wird das Gel z.B. auf einen Bandträger aufgebracht, so dass das vorgefertigte Haftband auf das Gestrick genäht oder geklebt werden kann.

Wie Fig. 2 zeigt, befindet sich der Gestricküberzug 3 proximal der obersten Dichtlippe 5a, so dass diese - anders als im Stand der Technik - folglich freiliegt und nicht vom Gestricküberzug 3 übergriffen ist. Sie kann folglich ihre Dichtfunktion komplett erfüllen, etwaige Durchmesserzunahmen infolge eines Übergriffs der Dichtlippe 5a durch den Gestricküberzug 3 sind, anders als im Stand der Technik, nicht gegeben.

Fig. 3 zeigt in Form einer vergrößerten Prinzipdarstellung eine Schnittansicht durch den "Haftbereich" zwischen Gestricküberzug 3 und Silikonliner 2. Ersichtlich liegt die Haftschicht 8 unmittelbar auf der Oberfläche 6 des Silikonliners 2 auf, sie geht einen adhäsiven Kontakt mit der Oberfläche 6 ein. Der Gestricküberzug 3 liegt über seine gesamte Länge flächig auf der Silikonlineroberfläche 6 auf, er ist etwas gedehnt, nachdem ja der innerhalb des Silikonliners befindliche Stumpf den Silikonliner 2 etwas dehnt. Diese Dehnung wird von der Haftschicht 8, die wie beschrieben aus einem Elastomer oder Elastomergel und vorzugsweise SEPS besteht, ohne weiteres mitvollzogen. Ersichtlich befindet sich das distale Ende 7 des Gestricküberzugs 3 proximal der obersten Dichtlippe 5a.

Fig. 4 zeigt in Form einer vergrößerten Detailansicht eine Prinzipdarstellung eines unteren, distalen Endes 7 des Gestricküberzugs 3. Der Gestricküberzug 3 besteht wie beschrieben aus einem Flachgestrick, exemplarisch ist der Strickfaden 9 und ein elastischer Faden 10, die das Gestrick bilden, dargestellt, wobei Fig. 4 natürlich nicht den tatsächlichen Fadenverlauf darstellt.

Ersichtlich ist das Gestrick im Bereich des distalen Endes 7 so gestrickt, dass sein Durchmesser von einer maximalen Gestrickdicke D auf eine reduzierte Dicke d abnimmt, so dass sich eine Art "Stufe" ausbildet. In dieser ist die Haftschicht 8 aufgebracht, so dass sich insgesamt auch im Bereich des distalen Endes 7 die Gesamtdicke D kaum bzw. nicht ändert.

Ersichtlich ist, wie durch die gestrichelte Linie 11 dargestellt, das Material der Haftschicht 8 in das Gestrickinnere, also in die einzelnen Maschen eingedrungen, was möglich ist, nachdem vorzugsweise das Haftschichtmaterial entweder im flüssigen Zustand durch Aufstreichen oder Aufgießen oder Ähnliches aufgebracht wird, mithin also das Gestrick benetzen kann und in das Maschenvolumen eindringen kann, oder nachdem es durch Aufschmelzen eines aufgelegten Materialbandes verflüssigt vorliegt und ebenfalls in das Gestrick respektive Maschenvolumen eindringen kann, wo es die Fäden 9, 10 benetzt und nach Aushärten respektive Vernetzen einen festen Verbund zum Gestrick eingeht.

Der Gestricküberzug 3 ist im Bereich seiner beiden offenen Enden so gestrickt, dass er sich nicht einrollen kann, das heißt, dass ein entsprechender oberer und unterer Strickbund gestrickt wird.

Neben einem Liner, bestehend vollständig oder nur an der Außenseite aus Silikon, mit einer Silikon- oder Gleitschichtoberfläche, wie er in den Figuren exemplarisch gezeigt ist, kann ein Liner auch eine Textilaußenhaut aufweisen. Auch auf dieser hält die Haftschicht aufgrund ihrer adhäsiven Eigenschaft und fixiert den Gestricküberzug 3 gegen ein Hochrutschen.

Ferner kann der Liner auch vollständig oder zumindest an der Außenseite anstelle des Silikons auch aus einem Polyurethtan bestehen, das gegebenenfalls mit einer, bei Bedarf auch nur lokal vorgesehenen, Gleitschicht oder einer Textilschicht belegt ist. Auch auf solchen Oberflächen bietet die beschriebene Haftschicht des erfindungsgemäßen Gestricküberzugs 3 eine sichere Fixierung des Gestricküberzugs 3.

## Patentansprüche

1. Anordnung zum Überziehen über einen Gliedmaßenstumpf zur Fixierung eines Prothesenschafts, umfassend einen über den Stumpf zu ziehenden Liner (2) und einen über diesen außenseitig zu ziehenden Gestricküberzug (3) zur Kompensation von Stumpfvolumenschwankungen, wobei der Liner (2) wenigstens eine im Bereich des distalen Endes ringförmig umlaufende Dichtlippe (5, 5a) zur dichten Anlage an der Innenseite des Prothesenschafts aufweist, **dadurch gekennzeichnet, dass** der Gestricküberzug (3) lediglich im Bereich seines distalen Endes (7) eine Haftschicht (8) aufweist, die adhäsiv mit der Lineroberfläche (6) zusammenwirkt.

2. Anordnung nach Anspruch 1, **dadurch gekennzeichnet, dass** der Liner eine äußere Oberfläche aus Silikon, gegebenenfalls mit einer aufgebrachten Gleitbeschichtung, oder aus einem aufgezogenen Textil aufweist.

3. Anordnung nach Anspruch 1 oder 2, **dadurch gekennzeichnet, dass** die Haftschicht (8) lediglich auf der Innenseite des Gestricküberzugs (3) aufgebracht ist, oder dass die Haftschicht (8) teilweise in das Gestrick eingedrungen ist.

4. Anordnung nach Anspruch 3, **dadurch gekennzeichnet, dass** die Haftschicht (8) in Form eines Haftbands aufgenäht oder aufgeklebt ist, oder dass die Haftschicht (8) auf das Gestrick aufgegossen, aufgestrichen oder auf dem Gestrick aufgeschmolzen und ausgehärtet ist.

5. Anordnung nach einem der vorangehenden Ansprüche, **dadurch gekennzeichnet, dass** die Haftschicht (8) aus einem dehnbaren Elastomer oder einem Elastomergel bestehend aus einem Elastomer und einem Weichmacheröl besteht.

6. Anordnung nach Anspruch 5, **dadurch gekennzeichnet, dass** das Elastomer ein Styrolblockcopolymer ist, insbesondere SEPS ist.

7. Anordnung nach einem der vorangehenden Ansprüche, **dadurch gekennzeichnet, dass** der Gestricküberzug (3) zumindest in dem Bereich, in dem die Haftschicht (8) aufgebracht ist, dünner gestrickt ist als im übrigen Gestrickbereich.

8. Anordnung nach einem der vorangehenden Ansprüche, **dadurch gekennzeichnet, dass** der Gestricküberzug (3) eine zylindrische oder konische Grundform aufweist, wobei insbesondere der Gestricküberzug (3) ein Flachgestrick ist.

9. Gestricküberzug, der für eine Anordnung nach einem der vorangehenden Ansprüche bestimmt ist, wobei lediglich im Bereich des distalen Endes (7) des Gestrickbezugs (3) eine Haftschicht (8) vorgesehen ist, die derart ausgelegt ist, dass sie adhäsiv mit einer Lineroberfläche (6) bestehend aus Silikon, einer, insbesondere auf der Silikonoberfläche aufgebrachten, Gleitbeschichtung oder einem textilen Überzug eines Liners (2) zusammenwirkt.

10. Gestricküberzug nach Anspruch 9, **dadurch gekennzeichnet, dass** die Haftschicht (8) lediglich auf der Innenseite des Gestricküberzugs (3) aufgebracht ist, oder dass die Haftschicht (8) teilweise in das Gestrick eingedrungen ist.

11. Gestricküberzug nach Anspruch 10, **dadurch gekennzeichnet, dass** die Haftschicht (8) in Form eines Haftbands aufgenäht oder aufgeklebt ist, oder dass die Haftschicht (8) auf das Textil aufgegossen, aufgestrichen oder auf dem Gestrick aufgeschmolzen und ausgehärtet ist.

12. Gestricküberzug nach einem der Ansprüche 9 bis 11, **dadurch gekennzeichnet, dass** die Haftschicht (8) aus einem dehnbaren Elastomer oder einem Elastomergel bestehend aus einem Elastomer und einem Weichmacheröl besteht.

13. Gestricküberzug nach Anspruch 12, **dadurch gekennzeichnet, dass** das Elastomer ein Styrolblockcopolymer ist, insbesondere SEPS.

14. Gestricküberzug nach einem der Ansprüche 9 bis 13, **dadurch gekennzeichnet, dass** er zumindest in dem Bereich, in dem die Haftschicht (8) aufgebracht ist, dünner gestrickt ist als im übrigen Gestrickbereich.

15. Gestricküberzug nach einem der Ansprüche 9 bis 14, **dadurch gekennzeichnet, dass** er eine zylindrische oder konische Grundform aufweist, wobei er insbesondere ein Flachgestrick ist.

16. Verfahren zur Herstellung eines Gestricküberzugs nach einem der Ansprüche 9 bis 15, **dadurch gekennzeichnet, dass** nach dem Stricken des Gestricks lediglich an einem Ende des Gestricks auf die Gestrickinnenseite eine adhäsive Haftschicht aus einem dehnbaren Elastomer oder einem Elastomergel bestehend aus einem Elastomer und einem Weichmacheröl in Form eines Haftbands aufgenäht oder aufgeklebt wird, oder durch Aufgießen, Aufstreichen oder Aufschmelzen und Aushärten mit dem Gestrick verbunden wird, wobei das Elastomer ein Styrolblockcopolymer, insbesondere SEPS, ist.

17. Verfahren nach Anspruch 16, **dadurch gekennzeichnet, dass** das Elastomer oder Elastomergel in Form eines Bands auf das Flachgestrick aufgebacht und, vorzugsweise mittels Hochfrequenz- oder Ultraschallbeheizung oder mittels geheizter Platten, erwärmt und aufgeschmolzen wird, so dass es das Flachgestrick benetzt, wonach es abkühlt und aushärtet.

18. Verfahren nach einem der Ansprüche 16 oder 17, **dadurch gekennzeichnet, dass** das Flachgestrick zumindest in dem Bereich, in dem die Haftschicht aufgebracht ist, dünner gestrickt wird als im übrigen Gestrickbereich.

19. Verfahren nach nach einem der Ansprüche 16 bis 18, **dadurch gekennzeichnet, dass** das Flachgestrick derart gestrickt wird, das der Gestricküberzug eine zylindrische oder konische Grundform aufweist.

## Claims

1. Arrangement for drawing over a limb stump for the fixing of a prosthesis socket, comprising a liner (2) that is to be drawn over the stump and a knitted covering (3) that is drawn over the exterior thereof to compensate stump volume variations, where the liner (2) has at least one annular peripheral sealing lip (5, 5a) in the region of the distal end for close contact with the internal side of the prosthesis socket, **characterized in that** the knitted covering (3) has, only in the region of its distal end (7), an adhesive layer (8) which interacts adhesively with the liner surface (6).

2. Arrangement according to Claim 1, **characterized in that** the liner has an exterior surface made of silicone, optionally with an applied friction-reducing coating, or made of a textile covering.

3. Arrangement according to Claim 1 or 2, **characterized in that** the adhesive layer (8) has been applied only on the internal side of the knitted covering (3), or that the adhesive layer (8) has to some extent penetrated into the knitted fabric.

4. Arrangement according to Claim 3, **characterized in that** the adhesive layer (8) takes the form of an adhesive strip applied by stitching or by adhesion, or that the adhesive layer (8) has been cast or spread onto the knitted fabric, or has been melted on the knitted fabric and has been hardened.

5. Arrangement according to any of the preceding claims, **characterized in that** the adhesive layer (8) is composed of an extensible elastomer or of an elastomer gel composed of an elastomer and of a plasticizer oil.

6. Arrangement according to Claim 5, **characterized in that** the elastomer is a styrene block copolymer, in particular SEPS.

7. Arrangement according to any of the preceding claims, **characterized in that** at least in the region in which the adhesive layer (8) has been applied the knitted covering (3) has been more thinly knitted than in the remaining region of the knitted fabric.

8. Arrangement according to any of the preceding claims, **characterized in that** the basic shape of the knitted covering (3) is cylindrical or conical, and in particular here the knitted covering (3) is a flat knitted fabric.

9. Knitted covering intended for an arrangement according to any of the preceding claims, with an adhesive layer (8) provided only in the region of the distal end (7) of the knitted covering (3), the said adhesive layer being designed in such a way that it interacts adhesively with a liner surface (6) composed of silicone, a friction-reducing coating in particular applied on the silicone surface, or a textile covering of a liner (2).

10. Knitted covering according to Claim 9, **characterized in that** the adhesive layer (8) has been applied only on the internal side of the knitted covering (3), or that the adhesive layer (8) has to some extent penetrated into the knitted fabric.

11. Knitted covering according to Claim 10, **characterized in that** the adhesive layer (8) takes the form of an adhesive strip applied by stitching or by adhesion, or that the adhesive layer (8) has been cast or spread onto the textile, or has been melted on the knitted fabric and has been hardened.

12. Knitted covering according to any of claims 9 to 11, **characterized in that** the adhesive layer (8) is composed of an extensible elastomer or of an elastomer gel composed of an elastomer and of a plasticizer oil.

13. Knitted covering according to Claim 12, **characterized in that** the elastomer is a styrene block copolymer, in particular SEPS.

14. Knitted covering according to any of Claims 9 to 13, **characterized in that** at least in the region in which the adhesive layer (8) has been applied it has been more thinly knitted than in the remaining region of the knitted fabric.

15. Knitted covering according to any of Claims 9 to 14, **characterized in that** its basic shape is cylindrical or conical, and in particular here it is a flat knitted fabric.

16. Process for the production of a knitted covering according to any of Claims 9 to 15, **characterized in that**, after the knitting of the knitted fabric, only at one end of the knitted fabric, an adhesive layer made of an extensible elastomer or of an elastomer gel composed of an elastomer and of a plasticizer oil is applied in the form of an adhesive strip by stitching or adhesion to the internal side of the knitted fabric, or is bonded to the knitted fabric via casting, spreading or melting and hardening, where the elastomer is a styrene block copolymer, in particular SEPS.

17. Process according to Claim 16, **characterized in that** the elastomer or elastomer gel is applied in the form of a strip to the flat knitted fabric and preferably heated and melted by means of highfrequency heating or ultrasound heating or by means of heated plates, in such a way that it wets the flat knitted fabric, and after that it cools and hardens.

18. Process according to Claim 16 or 17, **characterized in that** at least in the region in which the adhesive layer has been applied that the flat knitted fabric has been more thinly knitted than in the remaining region of the knitted fabric.

19. Process according to any of Claims 16 to 18, **characterized in that** the flat knitted fabric is knitted in such a way that the basic shape of the knitted covering is cylindrical or conical.

## Revendications

1. Système de recouvrement d'un manchon de réception de moignon pour la fixation d'une tige de prothèse, comprenant une doublure (2) devant être tirée par-dessus le moignon et un recouvrement tricoté (3) devant être tiré par-dessus l'extérieur de celle-ci pour compenser les variations de volume du moignon, la doublure (2) présentant au moins une lèvre d'étanchéité (5, 5a) de forme annulaire s'étendant dans la région de l'extrémité distale sur la périphérie pour l'application hermétique contre le côté intérieur de la tige de prothèse, **caractérisé en ce que** le recouvrement tricoté (3) présente une couche d'adhésion (8) uniquement dans la région de son extrémité distale (7), laquelle coopère par adhésion avec la surface de la doublure (6).

2. Système selon la revendication 1, **caractérisé en ce que** la doublure présente une surface extérieure en silicone, éventuellement avec un revêtement lubrifiant appliqué, ou un textile appliqué par-dessus.

3. Système selon la revendication 1 ou 2, **caractérisé en ce que** la couche d'adhésion (8) est appliquée uniquement sur le côté intérieur du recouvrement tricoté (3) ou **en ce que** la couche d'adhésion (8) est partiellement incorporée dans le tricot.

4. Système selon la revendication 3, **caractérisé en ce que** la couche d'adhésion (8) est cousue ou collée sous forme de bande d'adhésion, ou **en ce que** la couche d'adhésion (8) est coulée sur le tricot, enduite ou fondue sur le tricot puis durcie.

5. Système selon l'une quelconque des revendications précédentes, **caractérisé en ce que** la couche d'adhésion (8) se compose d'un élastomère extensible ou d'un gel élastomère constitué d'un élastomère et d'une huile plastifiante.

6. Système selon la revendication 5, **caractérisé en ce que** l'élastomère est un copolymère à blocs de styrène, en particulier du SEPS.

7. Système selon l'une quelconque des revendications précédentes, **caractérisé en ce que** le recouvrement tricoté (3), au moins dans la région dans laquelle la couche d'adhésion (8) est appliquée, est tricoté avec une moindre épaisseur que dans le reste de la région tricotée.

8. Système selon l'une quelconque des revendications précédentes, **caractérisé en ce que** le recouvrement tricoté (3) présente une forme de base cylindrique ou conique, le recouvrement tricoté (3) étant notamment un tricot plat.

9. Recouvrement tricoté, prévu pour un système selon l'une quelconque des revendications précédentes, une couche d'adhésion (8) étant prévue uniquement dans la région d'extrémité distale (7) du recouvrement tricoté (3), laquelle est conçue de telle sorte qu'elle coopère par adhésion avec une surface de doublure (6) constituée de silicone, un revêtement lubrifiant appliqué notamment sur la surface de silicone ou un recouvrement textile d'une doublure (2).

10. Recouvrement tricoté selon la revendication 9, **caractérisé en ce que** la couche d'adhésion (8) est appliquée uniquement sur le côté intérieur du recouvrement tricoté (3) ou **en ce que** la couche d'adhésion (8) est en partie incorporée dans le tricot.

11. Recouvrement tricoté selon la revendication 10, **caractérisé en ce que** la couche d'adhésion (8) est cousue ou collée sous forme de bande d'adhésion, ou **en ce que** la couche d'adhésion (8) est coulée sur le textile, enduite ou fondue sur le tricot puis durcie.

12. Recouvrement tricoté selon l'une quelconque des revendications 9 à 11, **caractérisé en ce que** la couche d'adhésion (8) se compose d'un élastomère extensible ou d'un gel élastomère constitué d'un élastomère et d'une huile plastifiante.

13. Recouvrement tricoté selon la revendication 12, **caractérisé en ce que** l'élastomère est un copolymère à blocs de styrène, en particulier du SEPS.

14. Recouvrement tricoté selon l'une quelconque des revendications 9 à 13, **caractérisé en ce qu'**au moins dans la région dans laquelle la couche d'adhésion (8) est appliquée, celui-ci est tricoté avec une moindre épaisseur que dans le reste de la région tricotée.

15. Recouvrement tricoté selon l'une quelconque des revendications 9 à 14, **caractérisé en ce qu'**il présente une forme de base cylindrique ou conique, et **en ce qu'**il se présente notamment sous forme de tricot plat.

16. Procédé de fabrication d'un recouvrement tricoté selon l'une quelconque des revendications 9 à 15, **caractérisé en ce qu'**après le tricotage du tricot, une couche d'adhésion adhésive constituée d'un élastomère extensible ou d'un gel élastomère constitué d'un élastomère et d'une huile plastifiante est cousue ou collée sous forme de bande adhésive uniquement au niveau d'une extrémité du tricot sur le côté intérieur du tricot, ou est liée au tricot par coulage, enduction ou fusion et durcissement, l'élastomère étant un copolymère à blocs de styrène, en particulier du SEPS.

17. Procédé selon la revendication 16, **caractérisé en ce que** l'élastomère ou le gel d'élastomère est appliqué sur le tricot plat sous la forme de bande et est chauffé de préférence par un chauffage à haute fréquence ou à ultrasons ou au moyen de plaques chauffées et est fondu de telle sorte qu'il mouille le tricot plat, après quoi il est refroidi et durci.

18. Procédé selon l'une quelconque des revendications 16 ou 17, **caractérisé en ce que** le tricot plat, au moins dans la région dans laquelle la couche d'adhésion (8) est appliquée, est tricoté avec une moindre épaisseur que dans le reste de la région tricotée.

19. Procédé selon l'une quelconque des revendications 16 à 18, **caractérisé en ce que** le tricot plat est tricoté de telle sorte que le recouvrement tricoté présente une forme de base cylindrique ou conique.
